# FASCICULE DE BREVET EUROPEEN

(11) **EP 2 665 851 B1**
(45) Date de publication et mention de la délivrance du brevet: **23.09.2015**
(21) Numéro de dépôt: 12703579.8
(22) Date de dépôt: 16.01.2012
(51) Int. Cl.: D03D 15/00, A61N 5/06, G02B 6/00, D03D 1/00, D03D 13/00

(54) **TISSU LUMINEUX, DISPOSITIF D'ILLUMINATION DE LA PEAU, KIT ET PROCÉDÉ DE MISE EN OEUVRE**
BELEUCHTETES GEWEBE, VORRICHTUNG ZUR VERABREICHUNG VON LICHT AUF DIE HAUT, KIT UND VERFAHREN ZU SEINER VERWENDUNG
ILLUMINATED FABRIC, DEVICE FOR ADMINISTERING LIGHT TO THE SKIN, KIT AND METHOD FOR IMPLEMENTING SAME

(30) Priorité: 21.01.2011 FR 1100184
(43) Date de publication de la demande: 27.11.2013
(73) Titulaire: Laboratoires Clarins, 92200 Neuilly sur Seine (FR)
(72) Inventeur: COURTIN, Olivier, F-92200 Neuilly Sur Seine (FR)
(74) Mandataire: Bernstein, Claire Jacqueline
(86) Numéro de dépôt international: PCT/IB2012/050194
(87) Numéro de publication internationale: WO 2012/098488

(56) Documents cités:
- EP-A1- 1 815 048
- US-A- 5 568 964

## Description

L'invention se rapporte à un tissu lumineux, à un dispositif d'illumination de la peau, à un kit et à un procédé de mise en oeuvre.

Il existe de nombreux dispositifs de luminothérapie à usage professionnel, c'est-à-dire sous le contrôle d'un professionnel, tel qu'un médecin ou une infirmière. Cette restriction est due au fait que leur usage abusif ou dans des conditions inappropriées peut causer des dommages importants à l'utilisateur, allant de brûlures superficielles à un cancer de la peau en passant par des brûlures profondes du derme. L'utilisateur doit donc se rendre sur place pour son soin

De tels dispositifs sont généralement très encombrants en raison du nombre important de sources lumineuses nécessaires pour obtenir une illumination homogène et suffisamment puissante de la peau pour obtenir un effet thérapeutique de la lumière sur la peau.

Les sources lumineuses utilisées dans ces dispositifs professionnels sont généralement de deux types principaux : les tubes lumineux et les diodes lumineuses. A titre d'exemple, on peut citer l'appareil GentleWaves Pro Unit ou Aktilite CL128.

Il a été proposé des solutions permettant de réduire l'encombrement de ces dispositifs.

Par exemple, il a été proposé un masque en plastique rigide incorporant des diodes électroluminescentes. Cependant, ce dispositif est désagréable à porter et doit être adapté à chaque utilisateur en raison de sa morphologie et de la rigidité du masque. En outre, des tests ont montré que l'homogénéité de la lumière était très mauvaise : la lumière présente une intensité maximale au droit des diodes et perd de son intensité lorsque l'on s'éloigne des diodes. Pour améliorer l'homogénéité, une solution évidente consiste à augmenter le nombre de diodes. Néanmoins, on s'aperçoit que cette solution est beaucoup plus complexe qu'il n'y parait pour obtenir une homogénéité satisfaisante.

En outre, le système d'alimentation de chaque diode en électricité et le système d'évacuation de chaleur sont inesthétiques et restent encore rigides et trop encombrants.

Une autre solution envisagée à été de créer un tissu lumineux à base de fibres optiques dont l'une au moins des extrémités est reliée à une source lumineuse.

Un tel tissu est décrit dans le document WO2006/031350.

Ce tissu présente l'avantage d'être moins rigide que les masques en plastique, et d'être moins encombrant, car la source lumineuse peut être délocalisée et prend moins de place qu'un circuit de diode et son alimentation.

Néanmoins, des mesures ont montré que l'énergie lumineuse émise par de tels tissus est insuffisante et inhomogène.

Le document EP 1 815 048 vise à résoudre le problème de l'élasticité des fibres optiques. Il propose d'entourer la fibre optique avec un fil composite élastique. Ceci augmente encore plus le problème d'intensité et d'homogénéité lumineuse.

Pour remédier au problème de puissance, il a naturellement été proposé d'augmenter la puissance de la ou des sources lumineuses ou d'augmenter leur nombre.

Cependant, cela augmente la consommation d'énergie ainsi que l'encombrement.

En outre, il a été proposé d'éclairer les deux extrémités de chaque fibre. Cependant, chaque source reçoit, via les fibres optiques, de l'énergie lumineuse de l'autre source. Cela pose un problème important lorsqu'il s'agit de diodes lumineuses, en particulier de diodes laser, car elles risquent d'être rapidement et définitivement endommagées.

Pour remédier au problème d'homogénéité, il a été proposé d'augmenter le nombre de fibres optiques.

Cependant cela augmente le coût du dispositif, sa rigidité, sa fragilité, et n'améliore que très modestement l'homogénéité de la lumière émise.

Un premier objectif de la présente invention est de proposer un dispositif d'illumination de la peau économique, fiable, puissant, homogène, peu encombrant, agréable à porter et facilement adaptable à tout utilisateur, et suffisamment souple pour rester en contact avec la peau de l'utilisateur.

Par ailleurs, il a été observé que l'illumination de la peau pouvait avoir un effet esthétique anti âge et apaisant, en particulier sur le visage.

Pour faciliter l'utilisation de la lumière dans un but esthétique, il existe donc un besoin pour un dispositif d'illumination de la peau permettant un usage personnel, c'est-à-dire dont l'usage ne nécessite pas nécessairement le contrôle d'un professionnel. Grâce à un tel dispositif, l'utilisateur peut choisir de réaliser un soin esthétique à domicile, sans avoir à se déplacer dans un institut de beauté ou chez un médecin.

Un deuxième objectif de l'invention est de proposer un dispositif d'illumination de la peau à la fois efficace et sûr, c'est-à-dire sans danger en cas d'usage excessif ou inapproprié par l'utilisateur.

A cette fin, l'invention a pour objet un tissu lumineux présentant une direction transversale de trame et une direction longitudinale de chaîne, caractérisé en ce qu'il comprend longitudinalement une pluralité de groupes de huit fibres optiques consécutives tissées avec des fibres textiles, chaque groupe étant tissé transversalement selon un mélange d'armures Satin 4, d'armures Satin 6, éventuellement flottées, et d'armures Satin 8.

Selon d'autres modes de réalisation :
- le tissu lumineux peut comprendre, en outre, des fibres optiques supplémentaires tissées transversalement selon un mélange d'armures incomplètes Satin 4, Satin 6 et Satin 8 ;
- le tissu lumineux peut présenter les caractéristiques suivantes :
   - chaque groupe est tissé transversalement selon une alternance de motifs A constitués par une armure en Satin 8 et de motifs C constitués par quatre armures en Satin 4 disposées en carré, les motifs A et C étant systématiquement séparés par un motif B constitué par une armure en Satin 6, éventuellement flottée ; et
   - deux groupes successifs longitudinalement présentent des armures A et C alternées en opposition ;
- un huitième à un quart de l'armure en Satin 6 peut être constitué de lignes flottées dans lesquelles les fibres optiques ne sont pas tissées.
- le tissu lumineux peut présenter les caractéristiques suivantes :
   - le motif A présente une dimension transversale comprise entre 40 et 50 millimètres, avantageusement 44 millimètres ;
   - le motif B présente une dimension transversale comprise entre 25 et 35 millimètres, avantageusement 30 millimètres ;
   - le motif C présente une dimension transversale comprise entre 55 et 70 millimètres, avantageusement 62 millimètres ;
- le tissu lumineux peut présenter une densité de fibres optiques comprise entre 29 et 40 fibres optiques par cm ;
- les fibres optiques peuvent présenter un coeur en PMMA et une gaine en polymère fluoré ;
- la gaine peut présenter une épaisseur de 5µm, les fibres optiques présentant un diamètre total de 250 µm ;
- les fibres textiles peuvent être en un matériau choisi parmi le polyester, le coton, le lin, le polypropylène, les polyamides, ou un mélange de ceux-ci ;
- le tissu lumineux peut présenter une souplesse inférieure ou égale à 2000 mN, la souplesse étant définie comme étant la force nécessaire pour courber de 30° un échantillon de 3,5 cm x 5,0 cm.

L'invention se rapporte également à un dispositif d'illumination destiné à être disposé en contact avec de la peau d'un utilisateur, comprenant au moins une source lumineuse et un tissu lumineux selon l'invention précédente, dans lequel au moins une extrémité de chaque fibre optique est connectée à une source lumineuse.

Selon d'autres modes de réalisation :
- la source lumineuse peut présenter une longueur d'onde comprise entre 440 et 680 nm, de préférence entre 440 nm et 500nm, et/ou entre 590 nm et 680 nm ;
- le dispositif peut comprendre, en outre, un moyen de mémorisation de la durée et/ou de la fréquence d'utilisation et une minuterie apte à arrêter ou inhiber le fonctionnement de la ou des sources lumineuse(s) ;
- le dispositif peut comprendre, en outre, un moyen de lecture d'une information de fonctionnement du dispositif, le moyen de lecture étant en liaison fonctionnelle avec une unité centrale apte à commander ou inhiber le fonctionnement du dispositif en fonction de l'information de fonctionnement.

L'invention se rapporte également à un kit de traitement cosmétique de la peau, comprenant un dispositif selon l'invention précédente et au moins un contenant de produit cosmétique, le contenant portant une information de fonctionnement du dispositif.

Selon d'autres modes de réalisation :
- le contenant peut comprendre une puce RFID sur laquelle est mémorisée l'information d'utilisation, le moyen de lecture du dispositif étant un lecteur de puce RFID ;
- le contenant peut comprendre un code barre uni ou bidimensionnel représentant graphiquement l'information d'utilisation, le moyen de lecture du dispositif étant un lecteur de code barre uni ou bidimensionnel.

L'invention se rapporte également à un procédé de mise en oeuvre d'un kit selon l'invention précédente, le procédé comprenant les étapes suivantes :
- mettre en oeuvre le moyen de lecture pour lire l'information de fonctionnement et activer le dispositif conformément à cette information ;
- appliquer directement le tissu lumineux sur la peau d'un mammifère pour un traitement lumino-cosmétique de la peau du mammifère ;
une durée et un mode de fonctionnement de la source lumineuse étant automatiquement commandés en fonction de l'information de fonctionnement.

Selon d'autres modes de réalisation :
- le produit cosmétique peut comprendre au moins un actif pris dans le groupe constitué par : le Palmitoyl dipeptide 5 diaminohydroxybutyrate, de l'extrait de Cochlearia officinalis, un ferment de Thermus thermophillus, du Tiliroside, Pyroglutamylamylamidoethyl indole, une association Tiliroside/Palmitoyl dipeptide 5 diaminohydroxybutyrate , de l'extrait de Rhodiola rosea, de l'extrait de Spergularia rubra, de l'extrait de Camellia sinensis, de l'extrait de Lapsana communis, de l'extrait de Atractyloides lancea, de l'extrait de Cedrelopsis gravei, de l'extrait de Thymus citriodorus, de la Chrysine, de l'extrait de Dianthus carthusianorum, ou un mélange de ceux-ci ;
- le produit peut être appliqué avant ou après l'application du tissu lumineux sur la peau.

D'autres caractéristiques de l'invention seront énoncées dans la description détaillée ci-après, faite en référence aux figures annexées qui représentent, respectivement :
- la figure 1, une vue schématique en plan d'un tissu lumineux selon l'invention ;
- les figures 2 à 5, des représentations schématiques en plan d'armures, respectivement, de Satin 4, de Satin 6, de Satin 6 flotté et de Satin 8 ;
- les figures 2a à 5a, des vues schématiques partielle en coupe d'armures, respectivement, de Satin 4, de Satin 6, de Satin 6 flotté et de Satin 8 ;
- la figure 6, une représentation schématique en plan d'un premier mode de réalisation d'un ensemble tissé destiné à faire partie d'un tissu lumineux selon l'invention ;
- la figure 7, une représentation schématique en plan d'un deuxième mode de réalisation d'un ensemble tissé destiné à faire partie d'un tissu lumineux selon l'invention ;
- la figure 8, une représentation schématique en plan d'un troisième mode de réalisation d'un ensemble tissé destiné à faire partie d'un tissu lumineux selon l'invention ;
- la figure 9, une représentation schématique d'un premier mode de réalisation d'un dispositif lumineux selon l'invention ;
- les figures 10 et 11, des vues schématiques de face de deux modes de réalisation de connecteurs de fibre optique dans un dispositif lumineux selon l'invention ; et
- la figure 12, une représentation schématique d'un deuxième mode de réalisation d'un dispositif lumineux selon l'invention incorporé dans un kit comprenant un contenant de produit cosmétique.

L'invention se rapporte à un tissu lumineux et, en particulier, à un tissu lumineux destiné à être utilisé en application contre la peau d'un utilisateur.

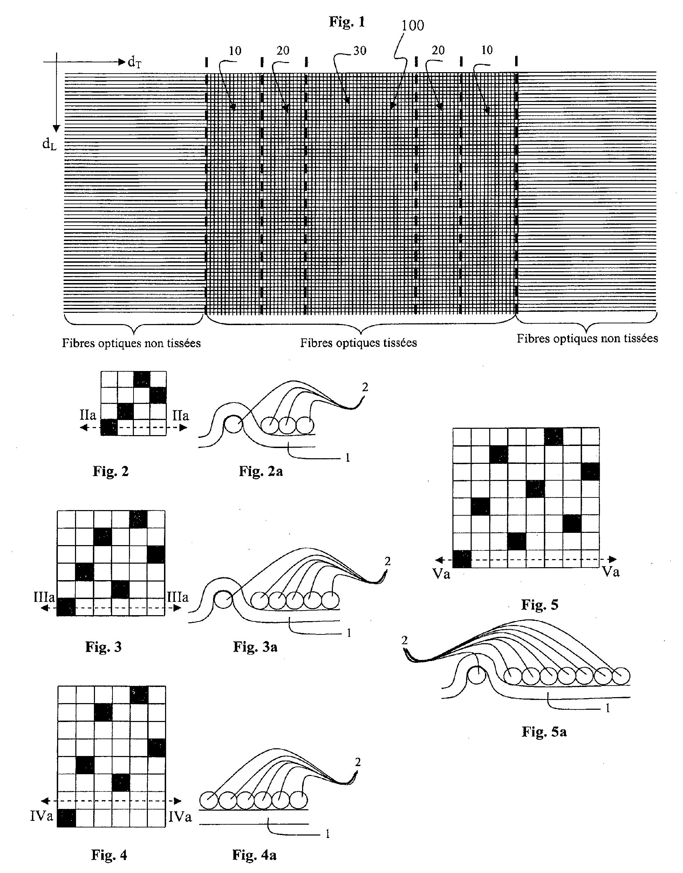

Ce tissu lumineux 100 est, comme illustré en figure 1, constitué, le long d'une direction longitudinale d_{L} du tissu, par des fils de chaîne en fibres textiles et, le long d'une direction transversale d_{T} du tissu, par des fils de trame en fibres optiques.

Selon la direction transversale d_{T}, le tissu lumineux selon l'invention comprend une alternance de bande 10 - 20 - 30 se distinguant par leur tissage.

Plus particulièrement, le tissu selon l'invention comprend longitudinalement une pluralité de groupes de huit fibres optiques consécutives tissées avec des fibres textiles, chaque groupe étant tissé transversalement selon un mélange d'armures Satin 4, d'armures Satin 6, éventuellement flottées, et d'armures Satin 8. La représentation de ces armures Satin 4, Satin 6, Satin 8 sont illustrées respectivement aux figures 2, 3 et 5.

Dans ce type de représentation bien connue de l'homme du métier, les cases blanches représentent les fibres textiles de chaîne alors que les cases noires représentent les fils de trame en fibre optique.

Une armure Satin 6 flottée signifie que certaines fibres optiques de l'armure Satin 6 ne sont pas tissées avec les fibres textiles. Une armure Satin 6 flottée est illustrée en figure 4 où l'on peut voir, par rapport à l'armure Satin 6 de la figure 3, deux lignes totalement blanches c'est-à-dire sans tissage de la fibre optique avec les fibres textiles.

Ainsi, au moins deux fibres optiques de chaque groupe de huit fibres optiques ne sont pas tissées dans l'armure en Satin 6 flottée et passent directement du motif A en Satin 8, respectivement C en Satin 4, vers le motif C en Satin 4, respectivement A en Satin 8.

Les figures 2a, 3a, 4a, et 5a illustrent respectivement les armures Satin 4, Satin 6, Satin 6 flottée et Satin 8 en coupe selon les flèches IIa-IIa, IIIa-IIIa, IVa-IVa et Va-Va.

Ainsi, dans l'armure en Satin 4 (figure 2a), la fibre optique 1 passe au dessus d'une fibre textile 2 (correspond à la case noire du damier de la figure 2) et en dessous de trois fibres textiles 2 (correspond aux cases blanches du damier de la figure 2).

Avantageusement, l'armure en Satin 4 utilisée présente un aspect rugueux grâce à un effet trame irrégulier (valeur de décochement vertical de +1 ; +2 ; -1 ; -2, voir Figure 2).

Dans l'armure en Satin 6 (figure 3a), la fibre optique 1 passe au dessus d'une fibre textile 2 (correspond à la case noire du damier de la figure 3) et en dessous de cinq fibres textiles 2 (correspond aux cases blanches du damier de la figure 3).

Avantageusement, l'armure en Satin 6 utilisée présente un aspect brillant, grâce à un effet trame irrégulier (valeur de décochement vertical de +2 ; +2 ; -3 ; +4 ; -2 ; -3 ; voir Figure 3).

Il en va de même pour l'armure en Satin 8 (figure 5a), où la fibre optique 1 passe au dessus d'une fibre textile 2 (correspond à la case noire du damier de la figure 5) et en dessous de sept fibres textiles 2 (correspond aux cases blanches du damier de la figure 5).

Avantageusement, l'armure en Satin 8 utilisée présente un aspect brillant, grâce à un effet trame avec décochement vertical de 3 (voir Figure 5).

Pour l'armure en Satin 6 flottée, la figure 4a illustre une coupe au niveau d'une ligne flottée. La fibre optique 1 passe donc sous toutes les fibres textiles 2, ce qui explique les cases blanches représentant la ligne flottée.

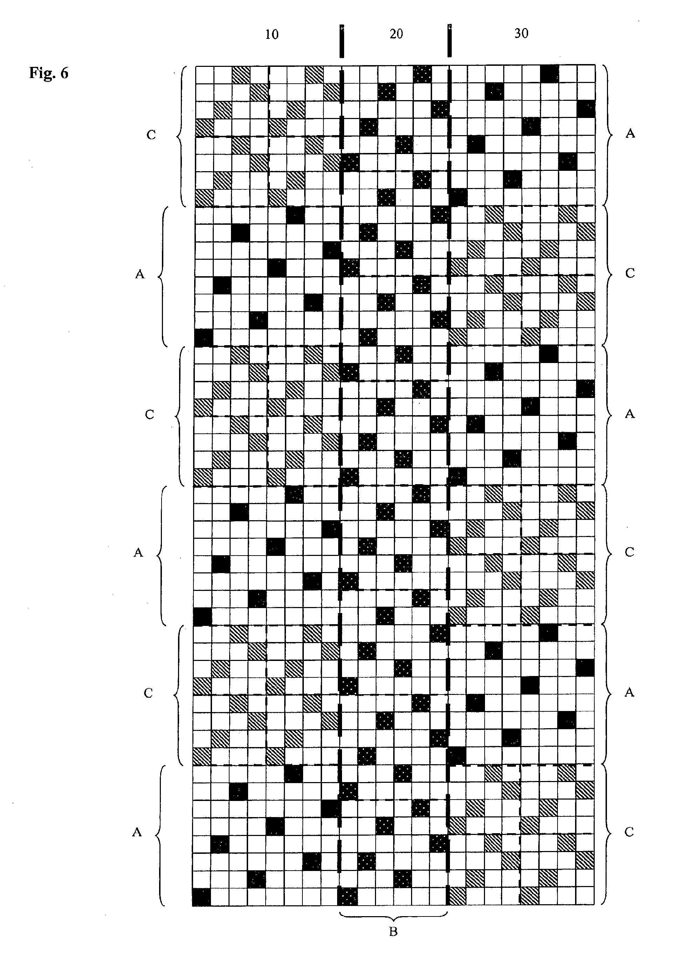

L'armure générale du tissu est présentée en figure 6, les motifs A, B et C étant remplis par, respectivement, satin de 4 effet trame irrégulier (valeur de décochement vertical de +1 ; +2 ; -1 ; -2), satin de 6 effet trame irrégulier (valeur de décochement vertical de +2 ; +2 ; -3 ; +4 ; -2 ; -3) et satin de 8 effet trame avec décochement vertical de 3. Pour cette armure, le rapport en trame est de 48 (plus petit produit commun entre une armure de rapport trame de 6 et une armure de rapport trame de 8).

En d'autres termes, le tissu lumineux conforme à l'invention comprend longitudinalement une pluralité de groupes de huit fibres optiques consécutives, chaque groupe étant tissé transversalement selon une alternance de motifs A constitués par une armure en Satin 8 et de motifs C constitués par quatre armures en Satin 4 disposées en carré, les motifs A et C étant systématiquement séparés par un motif B constitué par une armure en Satin 6, éventuellement flottées (noté B' par la suite).

En outre, longitudinalement, deux groupes successifs présentent des armures A et C alternées en opposition.

Bien entendu, le nombre d'armures A, B ou B' et C est calculé de préférence pour obtenir un tissu ayant une forme sensiblement rectangulaire. Autrement dit, pour tisser huit fibres optiques consécutives selon une armure en satin 4, il faut au moins deux répétitions selon la direction transversale d_{T} de l'armure en Satin 4 et deux répétitions selon la direction longitudinale d_{L}. Ceci est illustré en figure 6 où l'on peut voir, sur la bande 10, un premier motif C constitué par quatre armures en Satin 4, correspondant au tissage de huit fibres optiques, suivi, dans la direction longitudinale d_{L}, d'un motif A constitué par une armure en Satin 8.

Sur les figures 6 à 8, les lignes en pointillées servent uniquement à faciliter la distinction entre les différentes armures, et ne correspondent à aucune réalité physique sur le tissu lumineux selon l'invention. En outre, les cases représentant les fibres optiques apparentes ne sont pas toutes noires, comme elles auraient normalement dues être représentées selon la pratique habituelle, mais elles présentent des motifs différents uniquement destinés à faciliter l'identification des différentes armures et motifs. Ainsi, les fibres optiques tissées en Satin 4 sont illustrées par des cases hachurées, les fibres optiques tissées en Satin 6 sont illustrées par des cases noires à points blanc, et les fibres optiques tissées en Satin 8 sont illustrée par des cases noires.

Le tissu illustré à la figure 6 comprend donc un ensemble constitué par :
- une première bande 10 comprenant, dans la direction longitudinale d_{L}, trois motifs A constitués chacun d'une armure Satin 8 et trois motifs C constitués chacun de quatre armures Satin 4 disposées en carré, les motifs A et C étant alternés ;
- une deuxième bande 20 comprenant, dans la direction longitudinale d_{L}, huit motifs B constitués chacun d'une armure Satin 6 ; et
- une troisième bande 30 comprenant, dans la direction longitudinale d_{L}, trois motifs C constitués chacun de quatre armures Satin 4 disposées en carré et trois motifs A constitués chacun d'une armure Satin 8, les motifs C et A étant alternés. En outre, lorsque la première bande présente un motif A, la troisième bande présente, transversalement, un motif C. Les motifs A et C sont donc alternés en opposition.

Le tissu lumineux selon l'invention présente avantageusement plusieurs ensembles précédents dans la direction longitudinale d_{L}.

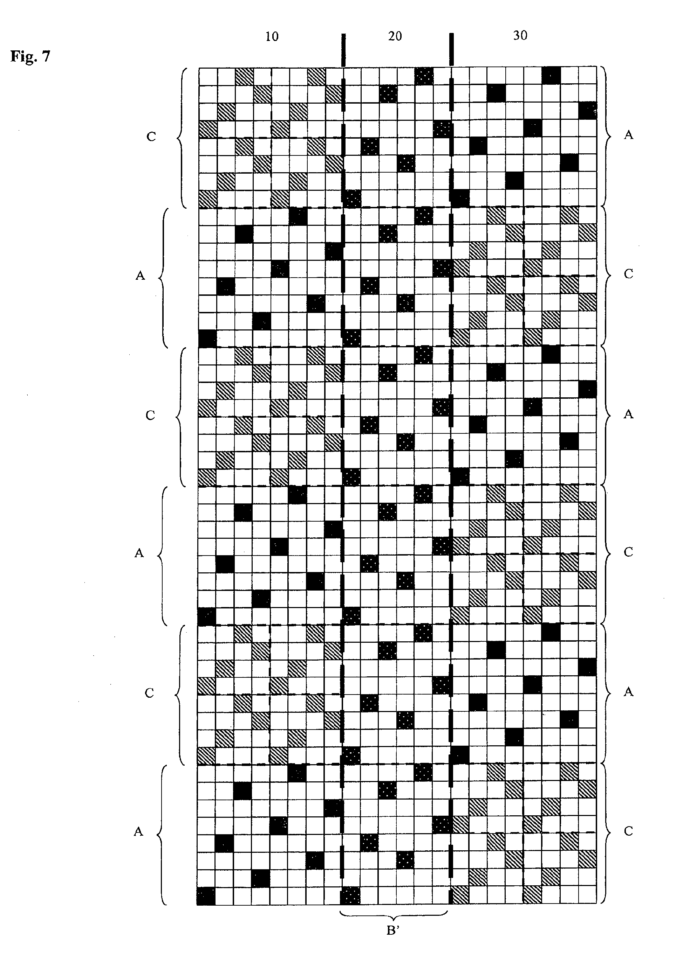

La figure 7 illustre une variante de l'ensemble de la figure 6, dans lequel la bande 20 comprend six motifs B' constitués chacun d'une armure Satin 6 flottée.

Les fils de chaîne utilisés sont, par exemple, en un fil blanc 100% Polyester, de 33 Tex composé de 288 filaments, produit par Sinterama sous la référence T371.

Les fils de trame utilisés sont en fibres optiques, par exemple, en Polyméthacrylate de méthyle (PMMA), de diamètre 250 µm (58 Tex), produites par Toray sous la référence PGR-FB250. Le diamètre cité comprend une gaine fluorée de 5 µm d'épaisseur.

La largeur (selon la direction transversale) des motifs dépend de la densité de fils de chaîne. La longueur (selon la direction longitudinale) des motifs dépend de la densité de fils de trame.

Dans l'exemple de réalisation, la densité de fils de chaîne est fixée à 20 fils/cm.

Différentes densités en trame ont été réalisées, allant de 29 fibres/cm à 40 fibres/cm (plafonnée pour des raisons géométriques). Les résultats les plus intéressants ont été obtenus pour des densités autour de 37 fibres/cm (35 à 40 fibres/cm).

Compte tenu de la morphologie et de la taille générale des visages, un tissu lumineux selon l'invention utilisé comme masque doit présenter une largeur (selon la direction transversale) supérieure ou égale à 200 mm sur le métier. Pour répondre à cette dimension et compte tenu de la densité de fils chaîne (20 fils/cm), le tissu selon l'invention comprend deux bandes 10 et 20 supplémentaires par rapport au mode de réalisation des figures 6 et 7.

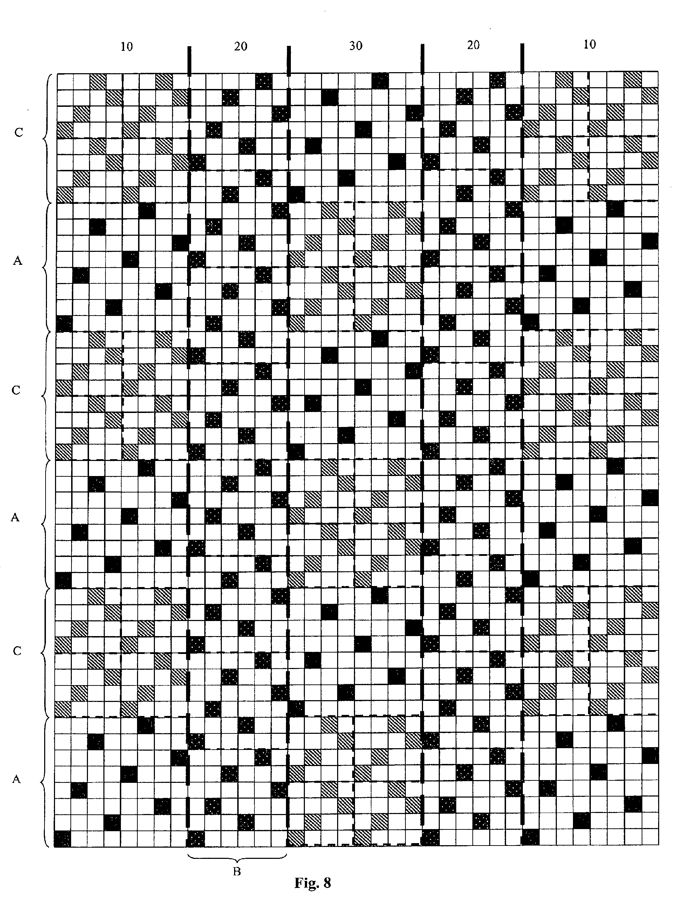

Les meilleurs résultats en termes d'homogénéité de la lumière diffusée sont obtenus avec un tissu comprenant au moins un ensemble illustré en figure 8 et constitué par :
- une première bande 10 comprenant, dans la direction longitudinale d_{L}, trois motifs A constitués chacun d'une armure Satin 8 et trois motifs C constitués chacun de quatre armures Satin 4 disposées en carré, les motifs A et C étant alternés ;
- une deuxième bande 20 comprenant, dans la direction longitudinale d_{L}, huit motifs B constitués chacun d'une armure Satin 6 ; et
- une troisième bande 30 comprenant, dans la direction longitudinale d_{L}, trois motifs C constitués chacun de quatre armures Satin 4 disposées en carré et trois motifs A constitués chacun d'une armure Satin 8, les motifs C et A étant alternés. En outre, lorsque la première bande présente un motif A, la troisième bande présente, transversalement, un motif C. Les motifs A et C sont donc alternés en opposition ;
- une quatrième bande 20 comprenant, dans la direction longitudinale d_{L}, huit motifs B constitués chacun d'une armure Satin 6 ; et
- une cinquième bande 10 comprenant, dans la direction longitudinale d_{L}, trois motifs A constitués chacun d'une armure Satin 8 et trois motifs C constitués chacun de quatre armures Satin 4 disposées en carré, les motifs A et C étant alternés comme dans la première bande.

Alternativement, les deuxième et quatrième bandes 20 peuvent comprendre des motifs B' constitués chacun d'une armure Satin 6 flottée.

Ainsi, chaque fibre optique alterne un tissage en Satin 4 ou en Satin 8 / un tissage en Satin 6 ou en Satin 6 flotté / un tissage en Satin 8 ou en Satin 4.

Cette séquence de bandes 10-20-30-20-10 permet d'obtenir à la fois un tissu lumineux pouvant être relativement large et souple, et une très bonne homogénéité de diffusion de la lumière.

Pour la réalisation d'un masque lumineux selon l'invention, on réalise un tissu dans lequel :
- le motif A présente une largeur (selon la direction transversale) comprise entre 40 et 50 millimètres, avantageusement 44 millimètres ;
- le motif B présente une largeur (selon la direction transversale) comprise entre 25 et 35 millimètres, avantageusement 30 millimètres ;
- le motif C présente une largeur (selon la direction transversale) comprise entre 55 et 70 millimètres, avantageusement 62 millimètres.

Ci-dessous sont indiquées (Tableau 1), pour chaque bande longitudinale, les largeurs, le nombre de fils du motif, le nombre de répétition du motif et le nombre total de fils.

| | Bande 10 | Bande 20 | Bande 30 | Bande 20 | Bande 10 | Total |
|---|---|---|---|---|---|---|
| Largeur (mm) | 44 | 30 | 64 | 30 | 44 | 212 |
| Nombre de fils du motif | 8 | 6 | 8 | 6 | 8 | |
| Nombre de répétitions du motif | 11 | 10 | 16 | 10 | 11 | |
| Nombre total de fils | 88 | 60 | 128 | 60 | 88 | 424 |

En tout, le tissu selon la figure 8 peut donc atteindre une dimension transversale d'une vingtaine de centimètres (21,2 dans l'exemple de réalisation précédent).

La largeur totale du tissu comprend 424 fils pour une laize de 212 mm sur le métier.

Le long des bords latéraux des bandes 10, deux fils tissés en point gaz sont introduits dans le but d'empêcher le dé-tissage de la structure.

Pour pouvoir être connectées à la source de lumière, les fibres optiques, introduites en trame, sont coupées à 290 mm environ des bords du tissu. Pour une manipulation aisée du produit à connecter, elles sont maintenues par un point toile (ou toutes autres armures) à 20 mm environ de leurs extrémités. La largeur totale du produit à connecter est de 792 mm environ.

Avantageusement, la densité de fibres optiques est comprise entre 29 et 40 fibres optiques par centimètre dans la direction longitudinale **d_{L},** de préférence 37 fibres/cm.

Pour réaliser un masque, on choisi de fabriquer un tissu lumineux ayant au moins une vingtaine de centimètres dans la direction longitudinale d_{L}. Plus précisément, dans l'exemple de réalisation décrit précédemment, le tissu présente une longueur de 22 cm.

Cela correspond à 816 duites en fibre optique soit 17 répétitions d'un rapport d'armure en trame de 48 duites (pour une densité de fibre optique de 37 fils/cm).

Avant et après le tissage des fibres optiques, un fil textile est avantageusement tissé pour empêcher le détissage des fibres optiques des extrémités et améliorer l'aspect visuel général du tissu.

Ce tissu a été obtenu produit en utilisant un peigne contenant 5 cellules par centimètre. Dans chacune des cellules, 4 fils sont introduits.

Au total 24 cadres ont été utilisés :
- 8 sont utilisés pour tisser les bandes longitudinales 10 (8 fils pour le motif répété 11 fois).
- 6 sont utilisés pour tisser les bandes longitudinales 20 (6 fils pour le motif répété 10 fois).
- 8 sont utilisés pour tisser la bande longitudinale 30 (8 fils pour le motif répété 16 fois).
- 2 sont utilisés pour réaliser le point toile (ou autre armure réalisable sur 2 cadres) servant au maintien des fibres optiques.

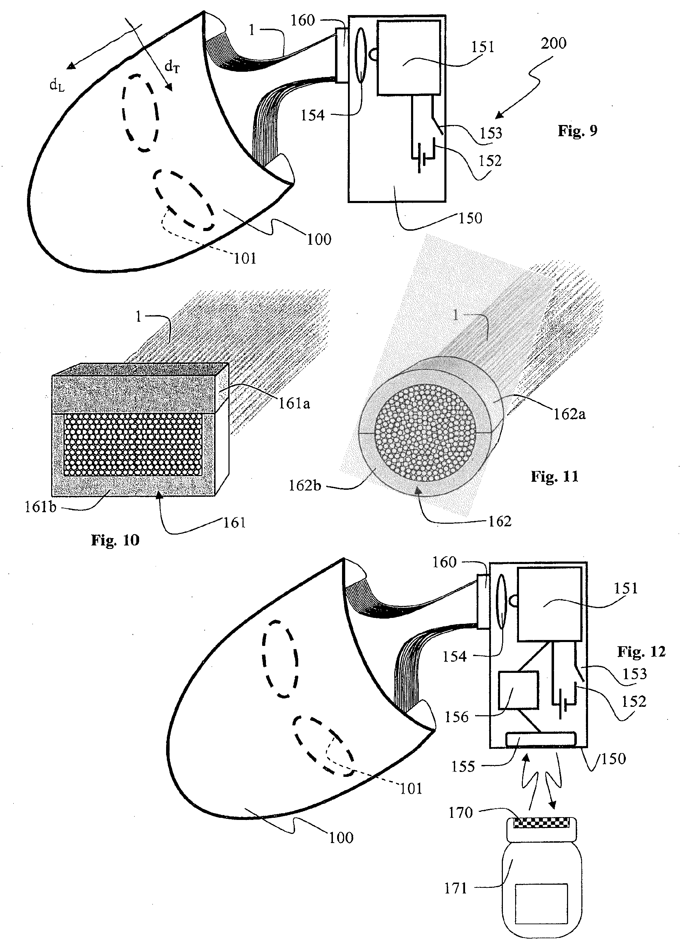

Une protection 101 (voir figures 9 et 12) des zones sensibles, tels que les yeux, est avantageusement prévue sur la face du tissu destinée à être disposée sur le visage. Cette protection doit être opaque aux longueurs d'ondes émises par le tissu lumineux.

L'homogénéité lumineuse du tissu selon l'invention est mesurée de la manière suivante.

Une source lumineuse est reliée aux deux extrémités de chaque fibre optique. Par exemple, on peut utiliser une diode électroluminescente (LED) délivrant une puissance lumineuse de 1,32 W à une longueur d'onde de 630 nm.

Ensuite une mesure de l'intensité lumineuse (IL) est réalisée sur une surface de 1 cm*1cm à l'aide d'un wattmètre. Par exemple, on peut utiliser un wattmètre avec une sonde de 1 cm² de marque Ophir et de modèle PD300. La mesure est mémorisée.

La mesure de l'IL est réitérée en plusieurs endroits sur le tissu lumineux, avantageusement sur toute la surface du tissu.

Les mesures sont ensuite comparées entre elles pour calculer l'écart moyen entre ces mesures, cet écart étant exprimé en pourcentage.

L'homogénéité est représentée par cet écart moyen.

Le tissu selon l'invention présente un écart moyen inférieur à 30% et sensiblement égal à 25% (à plus ou moins 3% près).

Le tissu lumineux selon l'invention présente également une très bonne souplesse permettant à la fois de le plier pour le ranger, et de l'étaler sur le visage pour qu'il le recouvre de manière relativement uniforme. Bien entendu, le tissu lumineux n'épouse pas complètement la forme du visage en raison des points saillants tels que le nez ou la bouche, mais il entre en contact avec la plupart des surfaces du visage. Il se distingue donc totalement des tissus lumineux encollés dans de la résine ou autre polymère, tels que les tissus prévus pour le traitement de la jaunisse du nourrisson et à plat, entre une table support et le nourrisson posé dessus.

Il n'existe pas de méthode normée pour la mesure de la souplesse d'un tissu. Afin de quantifier la souplesse du tissu obtenu selon l'invention, des mesures ont été réalisées avec une méthodologie utilisée pour mesurer la souplesse des cartons : il s'agit de mesurer la force (en newton) nécessaire pour courber l'échantillon (3,5 x 5,0 cm) d'un angle de 30°. Les résultats des mesures effectuées sur un échantillon de tissu conforme à l'invention montrent que la force nécessaire reste inférieure ou égale à 2 000 mN, y compris pour un nombre de fibres optique par centimètre supérieur à 30. Cette mesure a été réalisée avec une machine « Büchel stiffness tester ».

Grace au tissage selon l'invention, on obtient un tissu lumineux très souple, c'est-à-dire nécessitant une force inférieure ou égale à 2000 mN pour courber un échantillon de référence de 30°.

Avantageusement, les fibres optiques présentent un coeur en PMMA et une gaine en polymère fluoré. Avantageusement, les fibres optiques utilisées présentent une gaine ayant une épaisseur de 5µm, et ont un diamètre total (gaine + coeur) de 250 µm. Ce type de fibre donne d'excellents résultats de conduction de la lumière et de transmission latérale de la lumière grâce au tissage.

En effet, grâce au tissage selon l'invention, il est inutile de créer mécaniquement des défauts ou des imperfections dans la gaine pour faire sortir la lumière latéralement. Les fibres optiques selon l'invention sont donc dépourvues de tels défauts ou imperfections mais permettent, grâce au tissage particulier de l'invention, d'émettre de la lumière latéralement à travers la gaine, et de manière homogène de sorte que l'illumination est homogène.

En outre, le tissage selon l'invention évite que de la lumière émise à une extrémité soit introduite dans la source lumineuse à l'autre extrémité. Ainsi, la source lumineuse ne chauffe pas et sa durée de vie est optimisée.

Les fibres textiles utilisées peuvent être en différentes matières. Cependant, pour assurer une tenue et une résistance mécanique au tissu, tout en assurant sa souplesse et sa douceur (il est rappelé que ce tissu est destiné à être déposé sur le visage d'un utilisateur), les fibres textiles sont avantageusement choisies en polyester. D'autres matériaux choisis parmi le coton, le lin, le polypropylène, les polyamides, ou un mélange de ceux-ci, etc. peuvent également être utilisés.

Concernant la tension des fils de chaine, il faut trouver un juste milieu pour courber les fibres optiques et faire sortir la lumière tout au long des fibres sans que de la lumières ne soit réinjectée à l'extrémité opposée pour éviter de réintroduire de la lumière dans la ou les sources lumineuses. En effet, si lors du tissage, les fils de chaine ne sont pas assez tendus, de la lumière est transmise à l'extrémité opposée et risque d'endommager la source lumineuse. Si les fils de chaine sont trop tendus, les fibres optiques sont très courbées et la très grande majorité de la lumière risque de sortir par les bords du tissu alors que le centre du tissu n'émettra qu'une très faible lumière, affectant ainsi l'homogénéité de l'émission de lumière.

Les fibres optiques peuvent être regroupées selon des répartitions spatiales déterminées. Par exemple, les fibres destinées à recouvrir le pourtour de l'oeil sont regroupées ensembles, les fibres destinées à illuminer le front sont regroupées ensembles, etc. Le dispositif peut alors comprendre un moyen d'éclairage sélectif des extrémités des groupes de fibres pour illuminer ou non une ou plusieurs zones particulières, mais également pour moduler la puissance d'éclairement en fonction de la zone à éclairer en disposant, par exemple, des filtres entre la ou les sources lumineuses et les extrémités des groupes de fibres. Il est ainsi possible d'éclairer sélectivement et de manière contrôlée certaines zones par rapport à d'autres. Cependant, grâce au tissage selon l'invention, chaque zone présente une illumination homogène, même si la valeur de l'illumination peut varier d'une zone à l'autre de manière contrôlée.

Le tissu lumineux selon l'invention peut également comprendre des fibres optiques supplémentaires tissées transversalement selon un mélange d'armures incomplètes Satin 4, Satin 6 et Satin 8. Autrement dit, entre deux groupes transversaux de huit fibres optiques tissées selon des armures complètes de Satin 4, Satin 6 et Satin 8, le tissu peut comprendre des armures incomplètes, dans le sens longitudinal, de Satin 4, Satin 6 et Satin 8.

Le tissu lumineux selon l'invention est avantageusement destiné à être intégré à un dispositif d'illumination de la peau d'un utilisateur, le dispositif comprenant au moins une source lumineuse et un tissu lumineux précédemment décrit selon l'invention, au moins une extrémité de chaque fibre optique étant connectée à une source lumineuse.

Avantageusement, pour limiter l'encombrement du dispositif, les deux extrémités de chaque fibre sont connectées à une même source lumineuse.

La source lumineuse présente avantageusement une longueur d'onde comprise entre 440 et 680 nm, de préférence entre 440 nm et 500 nm, par exemple pour pour le traitement de peaux dites « à imperfections » (peau grasse), et/ou entre 590 nm et 680 nm pour obtenir un effet anti âge et apaisant.

Il est ainsi avantageusement possible d'utiliser une combinaison de deux longueurs d'onde comme, par exemple, le rouge et l'infrarouge.

Un tel dispositif est illustré à la figure 9.

Sur cette figure, le dispositif 200 comprend un tissu 100 illustré sous forme d'un masque, dont les fibres optiques 1 sont reliées à un boîtier 150 muni d'une source lumineuse 151 de sorte que les extrémités de chaque fibre soient disposées en face de la source lumineuse 151. On peut également utiliser plusieurs sources lumineuses (comme des diodes) dans le même boîtier.

La source lumineuse est reliée électriquement à une source d'énergie 152 et à un interrupteur 153 pour commander la marche/arrêt de la source lumineuse 151. Celle-ci peut fonctionner en continu ou en mode pulsé, par exemple selon l'une des séquences suivantes :
- Cycles de 250 millisecondes en marche / 100 millisecondes en arrêt, 100 cycles pour 35 secondes d'exposition ; ou
- Cycles de 500 microsecondes en marche / 150 microsecondes en arrêt, quatre cycles par train, 750 microsecondes ou 100 millisecondes d'intervalles entre deux trains pour 1000 secondes d'exposition.

Une lentille optique 154 peut avantageusement être intercalée entre la source et les extrémités des fibres optiques pour concentrer la lumière sur lesdites extrémités et limiter les pertes de transmission lumineuse.

La liaison entre les extrémités des fibres optiques et le boitier 150 se fait par un connecteur 160 dont deux modes de réalisation 161 et 162 sont illustrés aux figures 10 et 11.

Ces deux modes de réalisation se distinguent par la forme donnée à l'ensemble des extrémités des fibres optiques regroupées : rectangulaire (figure 10) ou circulaire (figure 11). Cette dernière forme présente l'avantage de limiter les pertes de transmission lumineuse et de favoriser l'homogénéité de la transmission lumineuse par le tissu car toutes les extrémités sont illuminées de manière homogène et toute la lumière émise par la source 151 est envoyée aux fibres. La lentille 153 joue également un rôle important dans cette transmission lumineuse optimale.

Les connecteurs illustrés aux figures 10 et 11 présentent deux parties 161a-162a et 161b-162b qui forment des mâchoires enserrant les fibres optiques 1 de manière à limiter l'espace entre les fibres optiques 1.

Pour améliorer la transmission lumineuse, les extrémités des fibres optiques sont poncées.

Avantageusement, le dispositif selon l'invention comprend, en outre, un moyen de mémorisation de la durée et/ou de la fréquence d'utilisation et une minuterie apte à arrêter ou inhiber le fonctionnement de la ou des sources lumineuse(s).

Il est ainsi possible de limiter la durée et/ou la fréquence d'utilisation du dispositif d'illumination de la peau selon l'invention, ce qui permet une utilisation par le grand public, sans la surveillance d'un médecin.

Selon un mode de réalisation particulièrement avantageux, le dispositif selon l'invention fait parti d'un kit et comprend, en outre, un moyen de lecture 155 d'une information de fonctionnement 170 du dispositif, le moyen de lecture 155 étant en liaison fonctionnelle avec une unité centrale 156 apte à commander ou inhiber le fonctionnement du dispositif en fonction de l'information de fonctionnement.

L'unité centrale 156 peut incorporer le moyen de mémorisation et la minuterie.

L'information d'information 170 peut être portée par un contenant 171 de produit cosmétique, tel qu'un pot ou un tube, compris dans le kit. Cette information 170 doit être sous une forme lisible et interprétable automatiquement par le lecteur, par exemple, sous la forme d'un code barre uni ou bidimensionnel représentant graphiquement l'information d'utilisation, d'une puce RFID sur laquelle est mémorisée l'information d'utilisation, etc.

Le moyen de lecture 155 du dispositif est alors choisi parmi un lecteur de code barre uni ou bidimensionnel, un lecteur de puce RFID, etc.

Ce moyen de lecture 155 est relié à l'unité centrale 156 de manière à commander ou inhiber le fonctionnement du dispositif en fonction :
- de l'information lue sur le pot ou le tube de produit cosmétique ; et/ou
- de la durée d'utilisation du dispositif.

L'information peut être une autorisation ou une interdiction de fonctionnement, mais également une instruction de fonctionnement comprenant, par exemple, la durée d'exposition maximale, un fonctionnement en continue ou sous forme puisée, une répartition spatiale déterminée de la puissance lumineuse, etc.

Pour mettre en oeuvre le kit selon l'invention, on met en oeuvre les étapes suivantes :
- activer le moyen de lecture 155 pour lire l'information de fonctionnement 170 et activer le dispositif (source lumineuse et tissu lumineux) conformément à cette information. Cette activation se fait par l'intermédiaire de l'unité centrale 156 ;
- appliquer directement le tissu lumineux 100 sur la peau d'un mammifère pour un traitement lumino-cosmétique de la peau du mammifère ;
une durée et un mode de fonctionnement de la source lumineuse étant automatiquement commandés en fonction de l'information de fonctionnement.

Il peut également être prévu d'autres réglages du dispositif comme, par exemple, l'âge de l'utilisateur, son type de peau, la fréquence d'utilisation, etc.

Grâce au kit selon l'invention, il est possible de faire fonctionner le tissu lumineux de manière adaptée au produit cosmétique utilisé et à l'utilisateur. En outre, celui-ci ne peut pas utiliser le tissu lumineux de manière dangereuse, de sorte qu'une utilisation grand public est possible.

Avantageusement, le produit cosmétique comprend au moins un actif pris dans le groupe constitué par : le Palmitoyl dipeptide 5 diaminohydroxybutyrate, de l'extrait de Cochlearia officinalis, un ferment de Thermus thermophillus, du Tiliroside, Pyroglutamylamylamidoethyl indole, une association Tiliroside/Palmitoyl dipeptide 5 diaminohydroxybutyrate, de l'extrait de Rhodiola rosea, de l'extrait de Spergularia rubra, de l'extrait de Camellia sinensis, de l'extrait de Lapsana communis, de l'extrait de Atractyloides lancea, de l'extrait de Cedrelopsis gravei, de l'extrait de Thymus citriodorus, de la Chrysine, de l'extrait de Dianthus carthusianorum, ou un mélange de ceux-ci.

L'association Tiliroside/Palmitoyl dipeptide 5 diaminohydroxybutyrate présente un effet amélioré lorsque la peau du mammifère, typiquement un humain, est éclairée par le tissu lumineux selon l'invention, en particulier dans les longueurs d'onde de l'infra-rouge.

Le produit cosmétique peut être appliqué avant ou après l'application du tissu lumineux sur la peau.

Lorsqu'il est appliqué avant, cela permet d'augmenter l'effet du produit par l'influence de la lumière émise par le tissu lumineux selon l'invention.

Lorsqu'il est appliqué après l'application du tissu lumineux sur la peau, cela permet une meilleure absorption du produit par la peau de l'utilisateur préalablement éclairée.

Bien entendu, il est possible d'adapter les dimensions du tissu lumineux selon l'invention en fonction des zones cutanées à traiter. Il est ainsi possible de réaliser des patchs de dimension plus faibles que le tissu nécessaire à l'éclairement d'un visage, afin de traiter des zones plus ciblées.

## Revendications

1. Tissu lumineux présentant une direction transversale de trame et une direction longitudinale de chaine, **caractérisé en ce qu'**il comprend longitudinalement une pluralité de groupes de huit fibres optiques (1) consécutives tissées avec des fibres textiles (2), chaque groupe étant tissé transversalement selon un mélange d'armures Satin 4, d'armures Satin 6, éventuellement flottées, et d'armures Satin 8.

2. Tissu lumineux selon la revendication précédente, comprenant, en outre, des fibres optiques supplémentaires tissées transversalement selon un mélange d'armures incomplètes Satin 4, Satin 6 et Satin 8.

3. Tissu lumineux selon l'une quelconque des revendications 1 ou 2, dans lequel :
- chaque groupe est tissé transversalement selon une alternance de motifs A constitués par une armure en Satin 8 et de motifs C constitués par quatre armures en Satin 4 disposées en carré, les motifs A et C étant systématiquement séparés par un motif B constitué par une armure en Satin 6, éventuellement flottée ; et
- deux groupes successifs longitudinalement présentent des armures A et C alternées en opposition.

4. Tissu lumineux selon l'une quelconque des revendications 1 à 3, dans lequel un huitième à un quart de l'armure en Satin 6 est constitué de lignes flottées dans lesquelles les fibres optiques ne sont pas tissées.

5. Tissu lumineux selon l'une quelconque des revendications précédentes, dans lequel :
- le motif A présente une dimension transversale comprise entre 40 et 50 millimètres, avantageusement 44 millimètres ;
- le motif B présente une dimension transversale comprise entre 25 et 35 millimètres, avantageusement 30 millimètres ;
- le motif C présente une dimension transversale comprise entre 55 et 70 millimètres, avantageusement 62 millimètres.

6. Tissu lumineux selon l'une quelconque des revendications précédentes, présentant une densité de fibres optiques comprise entre 29 et 40 fibres optiques par cm.

7. Tissu lumineux selon l'une quelconque des revendications précédentes, dans lequel les fibres optiques présentent un coeur en PMMA et une gaine en polymère fluoré.

8. Tissu lumineux selon l'une quelconque des revendications précédentes, présentant une souplesse inférieure ou égale à 2000 mN, la souplesse étant définie comme étant la force nécessaire pour courber de 30° un échantillon de 3,5 cm x 5,0 cm.

9. Dispositif d'illumination destiné à être disposé en contact avec de la peau d'un utilisateur, comprenant au moins une source lumineuse et un tissu lumineux selon l'une quelconque des revendications 1 à 9, dans lequel au moins une extrémité de chaque fibre optique (1) est connectée à une source lumineuse (151).

10. Dispositif selon la revendication précédente, dans lequel la source lumineuse (151) présente une longueur d'onde comprise entre 440 et 680 nm, de préférence entre 440 nm et 500nm, et/ou entre 590 nm et 680 nm.

11. Dispositif selon l'une quelconque des revendications 9 ou 10, comprenant, en outre, un moyen de mémorisation de la durée et/ou de la fréquence d'utilisation et une minuterie apte à arrêter ou inhiber le fonctionnement de la ou des sources lumineuse(s).

12. Dispositif selon l'une quelconque des revendications 9 à 11, comprenant, en outre, un moyen de lecture (155) d'une information de fonctionnement (170) du dispositif, le moyen de lecture étant en liaison fonctionnelle avec une unité centrale (156) apte à commander ou inhiber le fonctionnement du dispositif en fonction de l'information de fonctionnement.

13. Kit de traitement cosmétique de la peau, **caractérisé en ce qu'**il comprend un dispositif selon l'une quelconque des revendications 9 à 12 et au moins un contenant (171) de produit cosmétique, le contenant portant une information de fonctionnement (170) du dispositif.

14. Kit selon la revendication précédente, dans lequel le contenant (171) comprend une puce RFID sur laquelle est mémorisée l'information d'utilisation, le moyen de lecture du dispositif étant un lecteur de puce RFID.

15. Kit selon la revendication 13, dans lequel le contenant (171) comprend un code barre uni ou bidimensionnel représentant graphiquement l'information d'utilisation, le moyen de lecture (155) du dispositif étant un lecteur de code barre uni ou bidimensionnel.

16. Procédé de mise en oeuvre d'un kit selon l'une quelconque des revendications 13 à 15, **caractérisé en ce qu'**il comprend les étapes suivantes :
- mettre en oeuvre le moyen de lecture pour lire l'information de fonctionnement et activer le dispositif conformément à cette information ;
- appliquer directement le tissu lumineux sur la peau d'un mammifère pour un traitement lumino-cosmétique de la peau du mammifère ;
une durée et un mode de fonctionnement de la source lumineuse étant automatiquement commandés en fonction de l'information de fonctionnement.

17. Procédé selon la revendication précédente, dans lequel le produit cosmétique comprend au moins un actif pris dans le groupe constitué par : le Palmitoyl dipeptide 5 diaminohydroxybutyrate, de l'extrait de Cochlearia officinalis, un ferment de Thermus thermophillus, du Tiliroside, Pyroglutamylamylamidoethyl indole, une association Tiliroside/Palmitoyl dipeptide 5 diaminohydroxybutyrate , de l'extrait de Rhodiola rosea, de l'extrait de Spergularia rubra, de l'extrait de Camellia sinensis, de l'extrait de Lapsana communis, de l'extrait de Atractyloides lancea, de l'extrait de Cedrelopsis gravei, de l'extrait de Thymus citriodorus, de la Chrysine, de l'extrait de Dianthus carthusianorum, ou un mélange de ceux-ci.

## Patentansprüche

1. Beleuchtetes Gewebe, das eine querverlaufende Schussrichtung und eine längsverlaufende Kettrichtung aufweist, **dadurch gekennzeichnet, dass** es in der Längsrichtung eine Mehrzahl von Gruppen von acht aufeinanderfolgenden, mit Textilfasern (2) verwobenen optischen Fasern (1) umfasst, wobei jede Gruppe in der Querrichtung in einer Mischung aus 4-bindigen Satinbindungen, 6-bindigen Satinbindungen, ggf mit Flottierung, und 8-bindigen Satinbindungen gewebt ist.

2. Beleuchtetes Gewebe nach dem vorhergehenden Anspruch, ferner umfassend zusätzliche optische Fasern, die in der Querrichtung in einer Mischung aus unvollständigen 4-bindigen, 6-bindigen und 8-bindigen Satinbindungen gewebt sind.

3. Beleuchtetes Gewebe nach einem der Ansprüche 1 oder 2, wobei:
- jede Gruppe in der Querrichtung abwechselnd aus Mustern A, die aus einer 8-bindigen Satinbindung bestehen, und Mustern C, die aus vier quadratisch angeordneten 4-bindingen Satinbindungen bestehen, gewebt ist, wobei die Muster A und C systematisch durch ein Muster B getrennt sind, das aus einer 6-bindigen Satinbindung, ggf. mit Flottierung, besteht; und
- zwei in der Längsrichtung aufeinanderfolgende Gruppen einander abwechselnd gegenüberliegende Bindungen A und C aufweisen.

4. Beleuchtetes Gewebe nach einem der Ansprüche 1 bis 3, wobei ein Achtel bis ein Viertel der 6-bindigen Satinbindung aus flottierenden Reihen besteht, in die keine optischen Fasern eingewoben sind.

5. Beleuchtetes Gewebe nach einem der vorhergehenden Ansprüche, wobei:
- das Muster A eine Querabmessung von 40 bis 50 Millimetern, vorteilhafterweise 44 Millimetern, aufweist;
- das Muster B eine Querabmessung von 25 bis 35 Millimetern, vorteilhafterweise 30 Millimetern, aufweist;
- das Muster C eine Querabmessung von 55 bis 70 Millimetern, vorteilhafterweise 62 Millimetern, aufweist.

6. Beleuchtetes Gewebe nach einem der vorhergehenden Ansprüche, das eine Dichte der optischen Fasern von 29 bis 40 optischen Fasern pro cm aufweist.

7. Beleuchtetes Gewebe nach einem der vorhergehenden Ansprüche, wobei die optischen Fasern einen Kern aus PMMA und einen Mantel aus einem Fluorpolymer aufweisen.

8. Beleuchtetes Gewebe nach einem der vorhergehenden Ansprüche, das eine Biegsamkeit kleiner oder gleich 2000 mN aufweist, wobei die Biegsamkeit als die Kraft definiert ist, die notwendig ist, um ein 3,5 cm x 5,0 cm großes Muster um 30° zu biegen.

9. Vorrichtung zur Lichtverabreichung, die dazu vorgesehen ist, mit der Haut eines Nutzers in Berührung zu stehen, und die mindestens eine Lichtquelle und ein beleuchtetes Gewebe nach einem der Ansprüche 1 bis 9 umfasst, wobei mindestens ein Ende jeder optischen Faser (1) mit einer Lichtquelle (151) verbunden ist.

10. Vorrichtung nach dem vorhergehenden Anspruch, wobei die Lichtquelle (151) eine Wellenlänge von 440 bis 680 nm, bevorzugt 440 nm bis 500 nm, und/oder 590 nm bis 680 nm aufweist.

11. Vorrichtung nach einem der Ansprüche 9 oder 10, ferner umfassend eine Einrichtung zum Speichern der Dauer und/oder der Häufigkeit der Benutzung und einen Zeitgeber, der zum Anhalten oder Verhindern des Betriebs der Lichtquelle(n) geeignet ist.

12. Vorrichtung nach einem der Ansprüche 9 bis 11, ferner umfassend eine Einrichtung (155) zum Lesen einer Betriebsinformation (170) der Vorrichtung, wobei die Leseeinrichtung mit einer Zentraleinheit (156) in Wirkverbindung steht, die zum Steuern oder Verhindern des Betriebs der Vorrichtung in Abhängigkeit von der Betriebsinformation geeignet ist.

13. Kit zur kosmetischen Hauptbehandlung, **dadurch gekennzeichnet, dass** es eine Vorrichtung nach einem der Ansprüche 9 bis 12 und mindestens einen Behälter (171) für ein Kosmetikprodukt umfasst, wobei der Behälter eine Betriebsinformation (170) der Vorrichtung trägt.

14. Kit nach dem vorhergehenden Anspruch, wobei der Behälter (171) einen RFID-Chip umfasst, auf dem die Nutzungsinformation gespeichert ist, wobei die Leseeinrichtung der Vorrichtung ein RFID-Chiplesegerät ist.

15. Kit nach Anspruch 13, wobei der Behälter (171) einen ein- oder zweidimensionalen Strichcode umfasst, der die Nutzungsinformation graphisch darstellt, wobei die Leseeinrichtung (155) der Vorrichtung ein Lesegerät für einen ein- oder zweidimensionalen Strichcode ist.

16. Verfahren zur Verwendung eines Kits nach einem der Ansprüche 13 bis 15, **dadurch gekennzeichnet, dass** es die folgenden Schritte umfasst:
- Verwenden der Leseeinrichtung zum Lesen der Betriebsinformation und Aktivieren der Vorrichtung in Übereinstimmung mit dieser Information;
- unmittelbares Aufbringen des beleuchteten Gewebes auf die Haut eines Säugetiers zur lichtkosmetischen Behandlung der Haut des Säugetiers;
wobei eine Betriebsdauer und ein Betriebsmodus der Lichtquelle in Abhängigkeit von der Betriebsinformation automatisch gesteuert werden.

17. Verfahren nach dem vorhergehenden Anspruch, wobei das kosmetische Produkt mindestens einen Wirkstoff umfasst, der ausgewählt ist aus der Gruppe bestehend aus: Palmitoyl-Dipeptid-5-Diaminohydroxybutyrat, Cochleariaofficinalis-Extrakt, Thermus-thermophillus-Ferment, Tilirosid, Pyroglutamylamylamidoethylindol, einer Tilirosid-/Palmitoyl-Dipeptid-5-Diaminohydroxybutyrat-Verbindung, Rhodiola-rosea-Extrakt, Spergulariarubra-Extrakt, Camellia-sinensis-Extrakt, Lapsana-communis-Extrakt, Atractyloides-lancea-Extrakt, Cedrelopsis-gravei-Extrakt, Thymus-citriodorus-Extrakt, Chrysin, Dianthus-carthusianorum-Extrakt, oder deren Mischung.

## Claims

1. Illuminated fabric having a transverse weft direction and a longitudinal warp direction, **characterised in that** it comprises, longitudinally, a plurality of groups of eight consecutive optical fibres (1) interwoven with textile fibres (2), each group being transversely interwoven based on a mixture of satin 4 weaves, satin 6 weaves, optionally with floats, and satin 8 weaves.

2. Illuminated fabric as claimed in the preceding claim, further comprising additional optical fibres transversely interwoven based on a mixture of incomplete satin 4, satin 6 and satin 8 weaves.

3. Illuminated fabric as claimed in any one of claims 1 or 2, wherein:
- each group is transversely interwoven in an alternation of patterns A comprising one satin 8 weave and patterns C comprising four satin 4 weaves arranged in a square, patterns A and C being systematically separated by a pattern B comprising one satin 6 weave, optionally with a float; and
- two longitudinally successive groups having weaves A and C alternated in opposition.

4. Illuminated fabric as claimed in any one of claims 1 to 3, wherein one eighth to one quarter of the satin 6 weave comprises float lines in which the optical fibres are not interwoven.

5. Illuminated fabric as claimed in any one of the preceding claims, wherein:
- pattern A has a transverse dimension of between 40 and 50 millimetres, advantageously 44 millimetres;
- pattern B has a transverse dimension of between 25 and 35 millimetres, advantageously 30 millimetres;
- pattern C has a transverse dimension of between 55 and 70 millimetres, advantageously 62 millimetres.

6. Illuminated fabric as claimed in any one of the preceding claims, having a density of optical fibres of between 29 and 40 optical fibres per cm.

7. Illuminated fabric as claimed in any one of the preceding claims, wherein the optical fibres have a core of PMMA and a sheath of fluorinated polymer.

8. Illuminated fabric as claimed in any one of the preceding claims, having a flexibility of less than or equal to 2000 mN, flexibility being defined as being the force needed to bend a 3.5 cm x 5.0 cm sample by 30°.

9. Light administering device designed to be placed in contact with the skin of a user, comprising at least one light source and an illuminated fabric as claimed in any one of claims 1 to 9, wherein at least one end of each optical fibre (1) is connected to a light source (151).

10. Device as claimed in the preceding claim, wherein the light source (151) has a wavelength of between 440 and 680 nm, preferably between 440 nm and 500 nm, and/or between 590 nm and 680 nm.

11. Device as claimed in any one of claims 9 or 10, further comprising a means for storing the duration and/or frequency of use and a timer capable of stopping or disabling operation of the light source(s).

12. Device as claimed in any one of claims 9 to 11, further comprising a means (155) for reading operating information (170) pertaining to the device, the reading means being functionally connected to a central unit (156) capable of controlling or disabling operation of the device depending on the operating information.

13. Cosmetic skin treatment kit, **characterised in that** it comprises a device as claimed in any one of claims 9 to 12 and at least one container (171) for a cosmetic product, the container bearing operating information (170) pertaining to the device.

14. Kit as claimed in the preceding claim, wherein the container (171) comprises an RFID chip on which usage information is stored, the reading means of the device being an RFID chip reader.

15. Kit as claimed in claim 13, wherein the container (171) comprises a one-dimensional or two-dimensional bar code graphically representing the usage information, the reading means (155) of the device being a one-dimensional or two-dimensional bar code reader.

16. Method of using a kit as claimed in any one of claims 13 to 15, **characterised in that** it comprises the following steps:
- operating the reading means to read the operating information and activate the device in accordance with this information;
- applying the illuminated fabric directly to the skin of a mammal for a lumino-cosmetic treatment of the mammal's skin;
a duration and operating mode of the light source being automatically controlled depending on the operating information.

17. Method as claimed in the preceding claim, wherein the cosmetic product comprises at least one active ingredient selected from the group comprising: palmitoyl dipeptide 5 diaminohydroxybutyrate, extract of Cochlearia officinalis, a ferment of Thermus thermophillus, tiliroside, pyroglutamylamylamidoethyl indole, a combination of tiliroside/palmitoyl dipeptide 5 diaminohydroxybutyrate, extract of Rhodiola rosea, extract of Spergularia rubra, extract of Camellia sinensis, extract of Lapsana communis, extract of Atractyloides lancea, extract of Cedrelopsis grevei, extract of Thymus citriodoris, chrysin, extract of Dianthus carthusianorum, or a mixture thereof.
